# EUROPÄISCHE PATENTSCHRIFT

(11) **EP 2 696 920 B1**
(45) Veröffentlichungstag und Bekanntmachung des Hinweises auf die Patenterteilung: **07.06.2017**
(21) Anmeldenummer: 12718594.0
(22) Anmeldetag: 13.04.2012
(51) Int. Cl.: A61M 5/48, A61M 5/30

(54) **VORRICHTUNG ZUM EINSPRITZEN EINES MEDIUMS IN ODER UNTER DIE HAUT MIT EINER SERIE VON DRUCKIMPULSEN**
DEVICE FOR INJECTING A MEDIUM INTO OR UNDER THE SKIN WITH A SERIES OF PRESSURE PULSES
DISPOSITIF POUR L'INJECTION D'UN MILIEU DANS OU SOUS LA PEAU AU MOYEN D'UNE SÉRIE D'IMPULSIONS DE PRESSION

(30) Priorität: 13.04.2011 DE 102011007314
(43) Veröffentlichungstag der Anmeldung: 19.02.2014
(73) Patentinhaber: Stockmar, Jürgen, 1030 Wien (AT)
(72) Erfinder: Stockmar, Jürgen, 1030 Wien (AT)
(74) Vertreter: Lang, Johannes
(86) Internationale Anmeldenummer: PCT/EP2012/001620
(87) Internationale Veröffentlichungsnummer: WO 2012/139774

(56) Entgegenhaltungen:
- WO-A1-2006/086719
- WO-A1-2010/115499
- WO-A2-2004/093818
- US-A1- 2002 116 021

## Beschreibung

Die Erfindung betrifft eine Vorrichtung zum Einspritzen eines Mediums in oder unter die Haut mit einer Serie von Druckimpulsen.

In der medizinischen Anwendung ist die direkte Einspritzung von Medien unter Druck unter die Haut ohne Verwendung von Injektionsnadeln bekannt und wird z.B. für bestimmte Impfungen angewendet. Diese Einspritzungen bringen Medien, z.B. Medikamente, mit einem einzigen hohen Druckimpuls unter die Haut ein.

Die Aufgabe der Erfindung ist die Bereitstellung einer Vorrichtung, die nicht mit einem einzigen hohen Druckimpuls, sondern mit einer Serie von Druckimpulsen Medien unter Druck in oder unter die Haut spritzt.

Diese Aufgabe wird durch die Vorrichtung gemäß Anspruch 1 gelöst. Vorteilhafte Ausgestaltungen finden sich in den abhängigen Ansprüchen.

Die Vorrichtung gemäß Anspruch 1 umfasst einen Vorratsbehälter für das Medium, eine Applikationseinheit zum Einspritzen des Mediums in oder unter die Haut, eine hydraulische Hochdruckpumpe zum Pumpen des Mediums aus dem Vorratsbehälter in die Applikationseinheit, und eine elektronische Regeleinheit, die geeignet ist, die Applikationseinheit so zu regeln, dass das Medium mit einer Serie von Druckimpulsen in oder unter die Haut eingespritzt wird.

Die vorliegende Erfindung macht die transdermale Einbringung von vorzugsweise flüssigen Medien in oder unter die Haut mit einer Serie von Druckimpulsen möglich. Damit können Medien, vorzugsweise Medikamente, flächig mit minimalen Läsionen, ohne die bisher nötigen mehrfachen Einstiche durch Injektionsnadeln, in bzw. unter die Haut eingebracht werden. Die Unterbrechungen während der Serie von Druckimpulsen stellt sicher, dass der Strahl des flüssigen Mediums keine Schnittverletzungen ("Wasserstrahlschneider") hervorruft.

Die Dauer einer Serie von Druckimpulsen kann wenige Millisekunden bis zu einigen Sekunden betragen. Die hier beschriebene Vorrichtung erlaubt die transdermale Einbringung von flüssigen Medien mit einem weiten Viskositätsbereich. Die flüssigen Medien können den Stoff selbst darstellen (z.B. Hyaluronsäuregel, Botulinumtoxin oder Eigenfett), einen Wirkstoff in Lösung, als Gemisch oder auch den Wirkstoff ungelöst als (Nano)-Partikel enthalten. Der Partikel kann auch ein Implantat sein. Entsprechend des Löslichkeitskoeffizienten in Körperflüssigkeiten geben die Medien nach Erreichen des Zielgewebes den oder die Wirkstoffe in den Organismus ab. Damit kann ein sog. controlled release System eingerichtet werden.

Die elektronische Regeleinheit kann weiterhin geeignet sein, die hydraulische Hochdruckpumpe und eines oder mehrere Druckventile so zu regeln, dass das Medium mit wählbaren Drücken in oder unter die Haut eingespritzt wird. Durch die Regelung des Systemdrucks kann folglich ein beliebiger konstanter Ausgangsdruck eingestellt werden, und durch die Regelung der Applikationseinheit kann die Serie von Druckimpulsen festgelegt werden. Eine Leitung zur Zuführung des Mediums von der Hochdruckpumpe zur Applikationseinheit kann gleichzeitig als Druckreservoir und/oder zum Ausgleich von Druckspitzen zur hydraulischen Schwingungsglättung verwendet werden, was die Anzahl der benötigten Komponenten der Vorrichtung reduziert. Die Applikationseinheit kann mindestens eine mechanisch-hydraulisch, elektrisch oder piezoelektrisch betätigbare Injektionsdüse umfassen.

Das Einspritzen des Mediums in oder unter die Haut ist sehr flexibel regelbar. Erstens kann über die elektronische Regeleinheit die Einspritzfrequenz und damit die eingespritzte Menge des Mediums pro Zeiteinheit geregelt werden. Zweitens kann über die Druckregelventile der Einspritzdruck und damit die Einspritztiefe des Mediums in oder unter die Haut geregelt werden. Drittens, kann über die Form der Injektionsdüse der Applikationseinheit ein möglichst tiefes oder ein möglichst breites Einspritzen des Mediums in oder unter die Haut erreicht werden. Viertens kann über die Auswahl auswechselbarer Applikationseinheiten und/oder die Injektionsdüsen mit verschiedenen Durchmessern die einzubringende Menge des Mediums pro Fläche geregelt werden. Die Regelung kann über eine Bedientaste am Griffstück und/oder einen Fußschalter erfolgen.

Die vorliegende Erfindung wird nachfolgend anhand bevorzugter Ausführungsformen unter Bezug auf die beigefügten Zeichnungen erläutert.
- Fig. 1: zeigt eine Vorrichtung zur Einbringung von Medien in oder unter die Haut.
- Fig. 2: zeigt eine Applikationseinheit für die Vorrichtung zur Einbringung von Medien in oder unter die Haut.
- Fig. 3 und Fig. 4: zeigen verschiedene Injektionsdüsen für die Vorrichtung zur Einbringung von Medien in oder unter die Haut.

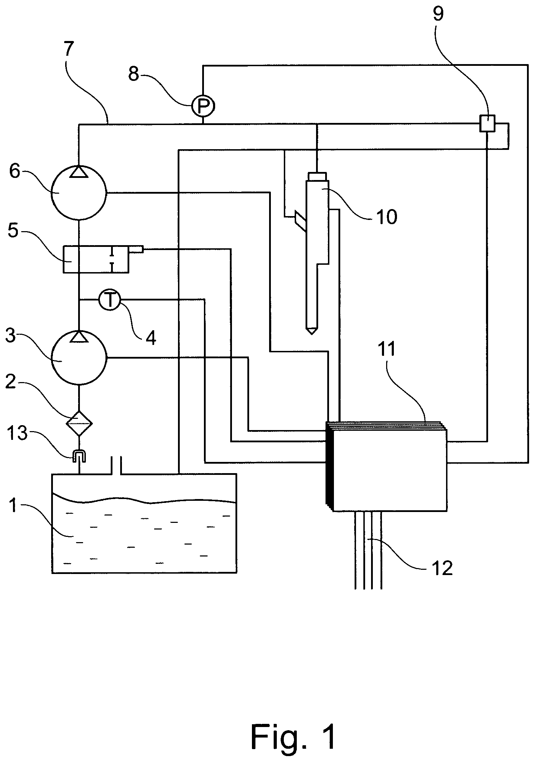

Fig. 1 zeigt mögliche Komponenten einer Vorrichtung zur Einbringung von Medien in oder unter die Haut:
1 Vorratsbehälter für die einzubringenden Medien
2 Filter
3 Vorförderpumpe
4 Temperatursensor für temperatursensible Medien
5 Druckregelventil
6 Hochdruckpumpe
7 Druckreservoir
8 Druckmesser
9 Druckbegrenzer mit Rücklaufleitung zum Vorratsbehälter
10 Applikationseinheit
11 Elektronische Regeleinheit
12 Leitungen für das Hand- und/oder Fußsteuergerät
13 Schnellkupplung

Eine beispielhafte Wirkungsweise der Vorrichtung zur Einbringung von Medien in oder unter die Haut wird im Folgenden in Verbindung mit Fig. 1 beschrieben. Aus einem Vorratsbehälter 1 saugt eine Vorförderpumpe 3 ein Medium über einen Filter 2 und drückt das Medium in eine Hochdruckpumpe 6. Die Hochdruckpumpe 6 ist vorzugsweise eine elektromotorisch angetriebene Radialkolbenpumpe mit einer Druck- und Mengenregelung. Ein Temperatursensor 4 kann die Temperatur des Mediums überwachen und bei temperatursensiblen Medien bei Überschreitung einstellbarer Temperaturgrenzen warnen. Ein Druckregelventil 5 regelt den Zuflussdruck zur elektronisch geregelten Hochdruckpumpe 6. Diese pumpt das Medium in ein Druckreservoir 7 zur Schwingungsglättung bzw. zum Druckausgleich, das vorzugsweise als Zuleitungsschlauch zur Zuführung des Mediums zu einer Injektionsdüse einer Applikationseinheit 10 dient. Ein vorgeschalteter Druckmesser 8 gibt das Drucksignal für die Regelung der Hochdruckpumpe 6 an die elektronische Regeleinheit 11 weiter. Die Vorrichtung kann weiterhin ein weiteres Druckregelventil bzw. einen Druckbegrenzer 9 mit Rücklaufleitung zum Vorratsbehälter 1 umfassen. Die elektronische Regeleinheit 11 umfasst die elektrische Energieversorgung der Vorrichtung und die Leitungen für das Handsteuergerät. Die elektronische Regeleinheit 11 kann folglich durch die Regelung der Hochdruckpumpe 6 und/oder der Druckregelventile 5, 9 einen beliebigen konstanten Ausgangsdruck einstellen, und durch die Regelung der Applikationseinheit 10 die Serie von Druckimpulsen in Bezug auf Höhe und Frequenz der Einspritzimpulse festgelegen. Eine Schnellkupplung 13 kann die leichte Ankopplung verschiedener Vorratsbehälter 1 zur Verwendung anderer Medien und einer geeigneten Spülflüssigkeit erlauben.

In anderen Worten, die beispielhafte Vorrichtung zur Einbringung von Medien in oder unter die Haut umfasst einen Vorratsbehälter 1 für die einzubringenden Medien, eine Regeleinheit 11 und eine Applikationseinheit 10, die durch ein Schlauchsystem bzw. ein elektrisches Kabel miteinander verbunden sind. Dies gestattet eine ergonomische Gestaltung der Applikationseinheit 10 z.B. in Form eines Kugelschreibers. Die Applikationseinheit 10 kann auch die Form einer endoskopischen Vorrichtung aufweisen, um Medien z.B. in die Magenwand einzubringen.

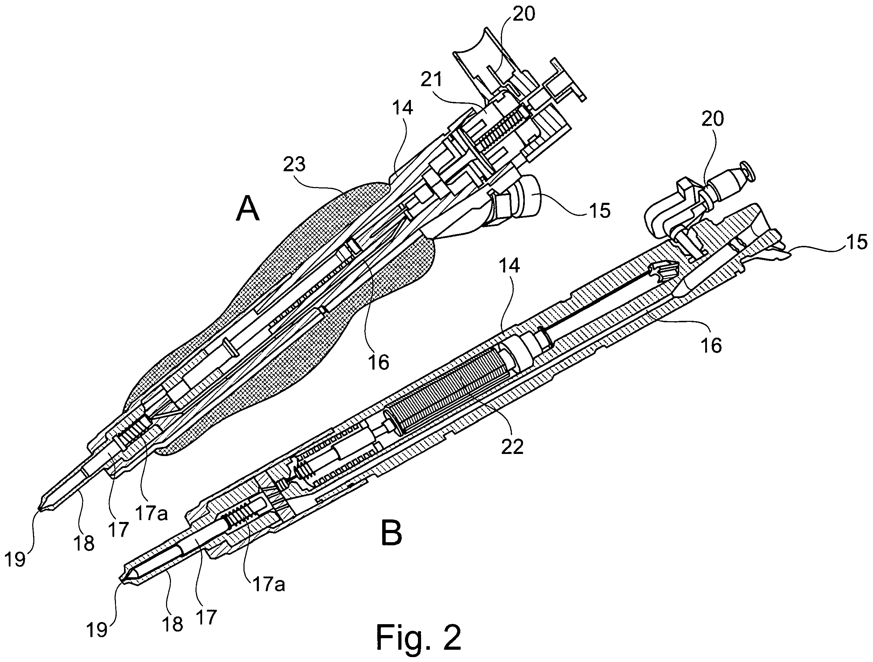

Fig. 2 zeigt mögliche Komponenten einer Applikationseinheit für die Vorrichtung zur Einbringung von Medien in oder unter die Haut:
14 Gehäuse
15 Medienzulauf
16 interner Medienkanal
17 Düsennadel
17a Feder
18 Düsenkopf
19 Düsenspitze mit Austrittsbohrungen
20 Anschluss für elektrische Steuerleitung
21 Hubmagnet
22 Piezo-Einheit
23 Griffstück

Eine beispielhafte Wirkungsweise der Applikationseinheit für die Vorrichtung zur Einbringung von Medien in oder unter die Haut wird im Folgenden in Verbindung mit Fig. 2 beschrieben. Ein Gehäuse 14 ist vorzugsweise von einem anatomisch geformten Handhalter umgeben, in das Bedientasten für Druck und Frequenz integriert sind. An dem Gehäuse 14 sind weiterhin Anschlüsse für einen Medienzulauf 15 durch Druckleitungen angebracht. Über einen internen Medienkanal 16 gelangt die Flüssigkeit zu einem vorzugsweise auswechselbaren Düsenkopf 18 mit mindestens einer Einspritzdüse. Dort verschließt eine Düsennadel 17, beaufschlagt von der Schließkraft einer Feder 17a, die Austrittsbohrungen in der Düsenspitze 19. Über elektrische Anschlüsse 20 erhält z.B. ein Hubmagnet 21 oder eine Piezo-Einheit 22 einen elektrischen Impuls und entlastet die Kraft auf die Düsennadel 17, welche sich hebt und die Austrittsbohrungen freigibt.

Die Injektionsdüse mit der federbelasteten Düsennadel 17 wird von der elektronischen Regeleinheit 11 geregelt und kann rein mechanisch-hydraulisch oder elektrisch gesteuert mit einem Hubmagneten (siehe Variante A in Fig. 2) oder mit piezoelektrischer Betätigung (siehe Variante B in Fig. 2) arbeiten. Ebenfalls ausgeführt werden können Lösungen des Einspritzvorgangs durch hydraulische Verstärkung oder Druckerzeugung durch piezoelektrischen Module in der Applikationseinheit 10. Die Vorzüge dieser Betätigung liegen in der hohen möglichen Grenzfrequenz und der feinen Modulierung der Druckverläufe beim Einspritzvorgang, die eine möglichst läsionsarme Durchdringung des jeweiligen Hauttypus erlauben. In anderen Worten, der Druck bzw. die Beschleunigung des Mediums sowie die Mediummenge sind variabel und präzise einstellbar über die elektrische Regeleinheit 11. Die Verteilung des Mediums in Bezug auf Tröpfchen- bzw. partikelgröße und Abstrahlwinkel kann durch das Design einer nachgeschalteten Düse bestimmt werden. Die Abgabe des Mediums kann in einzelnen Pulsen oder in Pulsserien mit regelbaren Frequenzen in einem weiten Frequenzband erfolgen.

Nach dem Auslösen der Betätigungstasten fährt der Bediener langsam mit der Applikationseinheit 10 über das zu behandelnde Gewebe. Je nach Bedarf kann die Applikationseinheit 10 auf die zu behandelnde Haut aufgesetzt oder mit einem manuell oder vermittels einer geeigneten Vorrichtung einstellbaren Abstand darüber positioniert werden. Je nach transdermal einzubringender Medienmenge müssen Frequenz und Bewegungsgeschwindigkeit sowie der ausgewählte Düsenkopf 18 koordiniert werden. Die gewünschte Eindringtiefe kann mit der Druckregelung und dem jeweiligen Düsenkopf 18 bestimmt werden.

Mit der Variation des Einspritzdruckes kann die Eindringtiefe in das Gewebe reguliert werden, während die Einspritzfrequenz und die gewählte Düseneinheit die einzubringende Menge pro Fläche bei gewählter Bewegungsgeschwindigkeit der Applikationseinheit über die behandelte Fläche bestimmen. Der Anwender kann also je nach gewünschter Eindringtiefe und Menge des Mediums in oder unter die Haut über die Einspritzfrequenz und die Wahl des Düsenkopfs 18 mit verschiedenen Düsenbohrungen die Menge und über die Druckregelung die Eindringtiefe des Mediums während der Applikation steuern.

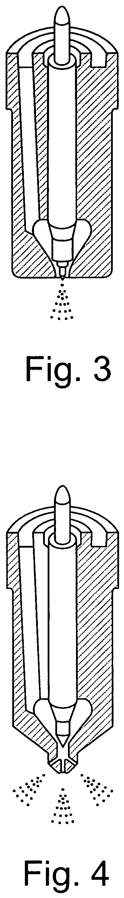

Je nach gewähltem Düsenkopf 18 kann der Injektionsstrahl scharf oder breiter gebündelt werden, d.h. als dünner Strahl oder als kegelförmiger Nebel im Mikrometerbereich. Die Wahl des auswechselbaren Düsenkopfes 18 bestimmt ebenfalls die Art der Einbringung (intradermal/intramukös oder transdermal/transmukös). Zapfendüsen (siehe Fig. 3) konzentrieren den Einspritzstrahl derart, dass eine möglichst tiefe Eindringung erreicht wird. Sie können einen zylindrischen oder einen kegelig aufgeweiteten Einspritzstrahl bilden. Lochdüsen (siehe Fig. 4) mit z.B. bis zu 12 Austrittsbohrungen hingegen erreichen eine breitere Verteilung des Mediums. Auch die Bohrungsdurchmesser der Düsenlöcher bestimmen als weiterer Parameter die Eigenschaften bzw. die Qualität der Einspritzung. Die Ausführung als dünner Strahl ist bevorzugt, wenn der Wirkstoff in tiefliegende Gewebe transdermal appliziert werden soll, z.B. in Muskulatur (Botulinumtoxin A). Die Ausführungsform als kegelförmiger Nebel wird bevorzugt bei flächenhafter Behandlung der Haut. Es können somit großflächige Hautareale mit dem Wirkstoff nahezu schmerzfrei infiltriert werden, z.B. die Epidermis oder die Dermis.

Zur einfachen Steuerung der Applikation werden die Betätigungen für Frequenz und Druck vorzugsweise direkt am Griffstück 23 in der Art integriert, dass eine Einhandbedienung möglich ist. Eine Variante dazu stellt eine Fußsteuerung dar, bei der Druck und Frequenz kombiniert in einem Pedal oder einzeln steuerbar sein können. Ebenfalls ist eine Kombination zwischen Hand- und Fußsteuerung möglich. Die Vorrichtung zur Einbringung von Medien kann ein Einmalartikel oder ein resterilisierbarer, wieder verwendbarer Artikel sein.

Je nach Dauer des elektrischen Impulses kann die Öffnungsdauer von wenigen Millisekunden bis zu einem Zeitraum von Sekunden reichen. Voraussetzung für eine lange Einspritzdauer ist eine ausreichend dimensionierte Leistung der Hochdruckpumpe 6. In Kombination mit der elektrischen Regeleinheit 11 wird eine feine Regulierung der transdermalen Einspritzung mit einer separaten Variation von Einspritzdruck und Einspritzfrequenz ermöglicht.

In Abhängigkeit von der zu injizierenden Flüssigkeit (korrodierend, nicht schmierend) können dafür korrosionsresistente Materialien oder spezielle Konstruktionsdetails notwendig werden.

Um eine Zerstörung von langkettigen Molekülen der einzubringenden Medien zu verhindern, können alle Ventile, Leitungsanschlüsse und Kanalleitungen mit möglichst großen Radien ohne scharfe Kanten ausgeführt werden.

## Patentansprüche

1. Vorrichtung zum Einspritzen eines Mediums in oder unter die Haut mit einer Serie von Druckimpulsen, umfassend
einen Vorratsbehälter (1) für das Medium,
eine Applikationseinheit (10) zum Einspritzen des Mediums in oder unter die Haut mit einer steuerbaren Einspritzdüse,
eine hydraulische Hochdruckpumpe (6) zum Pumpen des Mediums aus dem Vorratsbehälter (1) in die Applikationseinheit (10), und
eine elektronische Regeleinheit (11), die geeignet ist, die Applikationseinheit (10) so zu regeln, dass das Medium mit einer Serie von Druckimpulsen in oder unter die Haut eingespritzt wird,
wobei die Vorrichtung **dadurch gekennzeichnet ist, dass**
die Applikationseinheit (10) mindestens eine Injektionsdüse umfasst, die durch eine bewegliche Düsennadel (17) verschließbar und öffenbar ist.

2. Vorrichtung nach Anspruch 1, wobei die elektronische Regeleinheit (11) geeignet ist, die hydraulische Hochdruckpumpe (6) so zu regeln, dass das Medium mit wählbaren Drücken in oder unter die Haut eingespritzt wird.

3. Vorrichtung nach Anspruch 1 oder 2, wobei eine Leitung zur Zuführung des Mediums von der Hochdruckpumpe (6) zur Applikationseinheit (10) gleichzeitig als Druckreservoir (7) verwendbar ist.

4. Vorrichtung nach Anspruch 1, 2 oder 3, wobei die mindestens eine Injektionsdüse mechanisch-hydraulisch, elektromagnetisch oder piezoelektrisch betätigbar ist.

5. Vorrichtung nach einem der Ansprüche 1 bis 4, wobei die Injektionsdüse die Form einer Zapfendüse zum möglichst tiefen Einspritzen oder die Form einer Lochdüse zum möglichst breiten Einspritzen des Mediums in oder unter die Haut aufweist.

6. Vorrichtung nach einem der Ansprüche 1 bis 5, wobei die Applikationseinheit (10) und/oder die Injektionsdüse auswechselbar sind, sodass über die Auswahl verschiedener Applikationseinheiten (10) und/oder verschiedener Injektionsdüsen mit verschiedenen Durchmessern die einzubringende Menge des Mediums pro Fläche regelbar ist.

7. Vorrichtung nach einem der vorherigen Ansprüche, wobei über die elektronische Regeleinheit (11) die Einspritzfrequenz sowie das Einspritzprofil und damit die eingespritzte Menge des Mediums pro Zeiteinheit regelbar sind.

8. Vorrichtung nach einem der vorherigen Ansprüche, weiterhin umfassend Druckregelventile (5, 9), über die der Einspritzdruck und damit die Einspritztiefe des Mediums in oder unter die Haut durch die elektronische Regeleinheit (11) regelbar sind.

9. Vorrichtung nach einem der vorherigen Ansprüche, weiterhin umfassend eine Bedientaste am Griffstück (23) und/oder einen Fußschalter zur Regelung des Einspritzdrucks und/oder der Einspritzfrequenz des Mediums in oder unter die Haut.

10. Vorrichtung nach einem der vorherigen Ansprüche, weiterhin umfassend eine Schnellkupplung (13) für eine leichte Ankopplung verschiedener Vorratsbehälter (1) zur Verwendung verschiedener Medien und insbesondere zur Verwendung eines Spülmediums.

11. Vorrichtung nach einem der vorherigen Ansprüche, wobei die Applikationseinheit (10) räumlich von dem Vorratsbehälter (1) und der Hochdruckpumpe (6) getrennt ist.

## Claims

1. Device for injecting a medium into or underneath the skin with a series of pressure pulses, comprising:
a supply container (1) for the medium,
an application unit (10) for injecting the medium into or underneath the skin with a controllable injection nozzle,
a hydraulic high pressure pump (6) for pumping the medium from the supply container (1) into the application unit (10), and
an electronic control unit (11) configured to control the application unit (10) to inject the medium into or underneath the skin with a series of pressure pulses,
wherein the device is **characterized in that**
the application unit (10) comprises at least an injection nozzle which can be closed and opened by a movable nozzle needle (17).

2. The device according to claim 1, wherein the electronic control unit (11) is configured to control the hydraulic high pressure pump (6) to inject the medium with selectable pressures into or underneath the skin.

3. The device according to claims 1 or 2, wherein a line for supplying the medium from the high pressure pump (6) to the application unit (10) is simultaneously usable as a pressure reservoir (7).

4. The device according to claims 1, 2 or 3, wherein the at least one injection nozzle is mechanic-hydraulically, electromagnetically or piezoelectrically actuatable.

5. The device according to one of claims 1 to 4, wherein the injection nozzle is shaped as a pin nozzle for injecting as deep as possible or is shaped as a hole nozzle for injecting the medium as broad as possible.

6. The device according to one of claims 1 to 5, wherein the application unit (10) and/or the injection nozzle are exchangeable so that the amount of the medium to be injected per area can be controlled by selecting different application units (10) and/or different injection nozzles having different diameters.

7. The device according to one of the preceding claims, wherein the injection frequency as well as the injection profile and thereby the injected amount of the medium per unit of time is controllable via the electronic control unit (11).

8. The device according to one of the preceding claims, further comprising pressure valves (5, 9) via which the injection pressure and thereby the injection depth of the medium into or underneath the skin can be controlled by the electronic control unit (11).

9. The device according to one of the preceding claims, further comprising a control button at a handle (23) and/or a foot switch for controlling the injection pressure and/or the injection frequency of the medium into or underneath the skin.

10. The device according to one of the preceding claims, further comprising a quick coupling (13) for an easy coupling of different supply containers (1) in order to use different media and in particular to use a rinse medium.

11. The device according to one of the preceding claims, wherein the application unit (10) is spatially separated from the supply container (1) and the high pressure pump (6).

## Revendications

1. Dispositif d'injection d'un agent dans ou sous la peau avec une série d'impulsions de pression, comprenant un récipient de stockage (1) pour l'agent,
une unité d'application (10) pour l'injection de l'agent dans ou sous la peau avec une buse d'injection commandable,
une pompe haute pression (6) hydraulique pour le pompage de l'agent hors du récipient de stockage (1) dans l'unité d'application (10) et
une unité de régulation électronique (11) qui est appropriée afin de réguler l'unité d'application (10) de sorte que l'agent soit injecté avec une série d'impulsions de pression dans ou sous la peau,
dans lequel le dispositif est **caractérisé en ce que** l'unité d'application (10) comporte au moins une buse d'injection qui peut être fermée et ouverte par une aiguille de buse (17) mobile.

2. Dispositif selon la revendication 1, dans lequel l'unité de régulation électronique (11) est apte à réguler la pompe haute pression hydraulique (6) de sorte que l'agent soit injecté dans ou sous la peau avec des pressions sélectionnables.

3. Dispositif selon la revendication 1 ou 2, dans lequel une conduite pour l'amenée de l'agent de la pompe haute pression (6) à l'unité d'application (10) peut être utilisée en même temps comme réservoir de pression (7).

4. Dispositif selon la revendication 1, 2 ou 3, dans lequel la au moins une buse d'injection peut être actionnée par voie mécanohydraulique, électromagnétique ou piézoélectrique.

5. Dispositif selon l'une quelconque des revendications 1 à 4, dans lequel la buse d'injection présente la forme d'une buse d'injection à tenon pour une injection la plus profonde possible ou la forme d'une buse d'injection perforée pour une injection la plus large possible de l'agent dans ou sous la peau.

6. Dispositif selon l'une quelconque des revendications 1 à 5, dans lequel l'unité d'application (10) et/ou la buse d'injection sont interchangeables de sorte que la quantité d'agent par surface à introduire puisse être régulée par la sélection de différentes unités d'application (10) et/ou différentes buses d'injection avec différents diamètres.

7. Dispositif selon l'une quelconque des revendications précédentes, dans lequel la fréquence d'injection ainsi que le profil d'injection et ainsi la quantité injectée de l'agent par unité de temps peuvent être régulés par l'unité de régulation électronique (11).

8. Dispositif selon l'une quelconque des revendications précédentes, comprenant en outre des soupapes de régulation de pression (5, 9), par lesquelles la pression d'injection et ainsi la profondeur d'injection de l'agent dans ou sous la peau peuvent être régulées par l'unité de régulation électronique (11).

9. Dispositif selon l'une quelconque des revendications précédentes, comprenant en outre une touche de commande sur pièce de préhension (23) et/ou un commutateur au pied pour la régulation de la pression d'injection et/ou de la fréquence d'injection de l'agent dans ou sous la peau.

10. Dispositif selon l'une quelconque des revendications précédentes, comprenant en outre un coupleur rapide (13) pour un couplage aisé de différents récipients de stockage (1) pour pouvoir utiliser différents agents et en particulier pour pouvoir utiliser un agent de rinçage.

11. Dispositif selon l'une quelconque des revendications précédentes, dans lequel l'unité d'application (10) est spatialement séparée du récipient de stockage (1) et de la pompe haute pression (6).
